# DEMANDE DE BREVET EUROPEEN

(11) **EP 2 412 331 A1**
(43) Date de publication de la demande: **01.02.2012**
(21) Numéro de dépôt: 11174357.1
(22) Date de dépôt: 18.07.2011
(51) Int. Cl.: A61B 19/00, G01L 1/20

(54) **Dispositif de mesure d'un effort exercé par un fémur sur un tibia en vue d'une pose de prothèse articulaire**

(30) Priorité: 28.07.2010 FR 1056225
(71) Demandeur: AMPLITUDE Société à Responsabilité Limitée, 26000 Valence (FR)
(72) Inventeur: Ghestem, Didier, 62580 Neuville Saint Vaast (FR); Stahl, Philippe, 59262 Sainghin en Melantois (FR); Potel, Jean-François, 31500 Toulouse (FR); Boussaton, Michel, 31120 Portet sur Garonne (FR); Mailhe, Didier, 34980 Murles (FR); Rivat, Paul, 07130 Saint Peray (FR); Raguet, Marc, 51000 Chalons en Champagne (FR); LeTiec, Thierry, 11000 Carcassonne (FR); Francois, Jean-Marie, 67500 Marienthal (FR)
(74) Mandataire: Parzy, Benjamin Alain

(57) **Abrégé**

L'invention concerne un dispositif de mesure d'un effort exercé par un fémur sur un tibia en vue d'une pose de prothèse articulaire, le dispositif comportant un substrat (2) ayant une première face destinée à être posée sur une surface du tibia pour recouvrir ladite surface; un réseau d'entrée (3), un premier réseau de sortie (4) et un deuxième réseau de sortie (5) électriquement conducteurs, chaque réseau comportant des branches parallèles qui sont reliées entre elles à la manière d'un peigne, lesdits réseaux s'étendant sur une deuxième face du substrat opposée à la première face; un coussin (6) en matériau élastiquement déformable et électriquement conducteur recouvrant la deuxième face du substrat.

## Description

L'invention concerne un dispositif de mesure d'un effort exercé par un fémur sur un tibia en vue d'une pose de prothèse articulaire.

### ARRIERE PLAN TECHNOLOGIQUE DE L'INVENTION

Habituellement, lors d'une pose de prothèse de genou, le chirurgien réalise, à l'articulation du genou, une coupe du fémur et une coupe du tibia à l'aide d'un guide de coupe. Le chirurgien intercale alors une prothèse entre les parties coupées du fémur et du tibia. Le chirurgien doit s'assurer que ladite prothèse respecte à la fois l'anatomie initiale de l'articulation du genou ainsi que les tissus entourant ladite articulation et ce que le genou soit fléchi ou en extension.

A cette fin, le chirurgien commence par réaliser une coupe du plateau tibial pour insérer entre le tibia et le fémur un tenseur réglable permettant de déterminer précisément les dimensions de la prothèse adaptée au patient. Un dispositif de mesure est généralement inséré avec le tenseur entre le tibia et le fémur pour mesurer un effort exercé par le fémur sur le tibia.

En modifiant l'écartement entre le fémur et le tibia grâce au tenseur, le chirurgien peut ainsi relever l'effort exercé par le fémur sur le tibia pour différents angles de flexion du genou.

Il est ainsi connu du document US 2007/0219561 un dispositif de mesure d'un effort exercé par un fémur sur un tibia qui est intégré à un tenseur, celui-ci comportant un soufflet. Du fluide inséré dans le soufflet permet de monter ou d'abaisser le tenseur pour rapprocher ou écarter le fémur du tibia. En fonction de la pression du fluide à l'intérieur du soufflet, on peut ainsi déterminer l'effort exercé par le fémur sur le tibia. Toutefois, ce dispositif de mesure est complexe et délicat à mettre en oeuvre, en particulier si le fluide employé n'est pas de l'air. Il faut en effet s'assurer qu'en cas de fuite, l'articulation du genou ne sera pas infectée par le fluide.

Il est également connu du document WO 92/17113 un dispositif de mesure destiné à être agencé sous un tenseur qui écarte tibia et fémur. Le dispositif de mesure comporte :
- un substrat ayant une première face destinée à être posée sur une surface du tibia pour recouvrir ladite surface;
- quatre capteurs d'efforts disposés sur une deuxième face du substrat opposée à la première face de sorte que deux capteurs soient disposés sous un premier condyle du fémur et que deux capteurs soient disposés sous un deuxième condyle du fémur;
- une enveloppe englobant substrat et capteurs, l'enveloppe étant agencée de sorte à concentrer les efforts exercés par le fémur sur le tibia vers les capteurs.

Un tel dispositif de mesure s'avère compact mais encore complexe, le dispositif de mesure devant être conformé pour assurer une concentration des efforts vers les capteurs. En outre, huit fils sont nécessaires pour relier les quatre capteurs vers un organe de traitement de données.

Dans un domaine éloigné de la pose de prothèse de genou, il est connu du document US 5 510 812 de mesurer le déplacement d'un joystick dans un plan à l'aide d'un ou plusieurs capteurs comportant chacun :
- un substrat ;
- un réseau d'entrée et un réseau de sortie électriquement conducteurs, chaque réseau comportant des branches parallèles qui sont reliées entre elles à la manière d'un peigne, lesdits réseaux s'étendant sur une deuxième face du substrat opposée à la première face de sorte que les branches du réseau d'entrée s'étendent en alternance avec les branches du réseau de sortie;

- un coussin en matériau élastiquement déformable et électriquement conducteur recouvrant la deuxième face du substrat ;
- une plaque rigide en matériau isolant recouvrant ledit coussin.

En appliquant une force sur la plaque, on déforme le coussin qui en se compressant favorise la circulation d'un courant électrique entre les branches du réseau d'entrée et les branches du réseau de sortie.

L'homme du métier peut envisager de remplacer les quatre capteurs d'efforts du document WO 92/17113 par quatre capteurs identiques à ceux utilisés dans le joystick. Il est possible de simplifier un tel dispositif en ne recourant qu'à deux capteurs au lieu de quatre.

Toutefois, le dispositif s'avère peu compact. De plus, quatre fils sont encore nécessaires pour relier les deux capteurs vers un organe de traitement de données.

### OBJET DE L'INVENTION

L'objectif de l'invention est de proposer un dispositif de mesure compact d'un effort exercé par un fémur sur un tibia qui soit simplifié par rapport aux dispositifs de l'art antérieur.

### BREVE DESCRIPTION DE L'INVENTION

En vue de la réalisation de ce but, on propose un dispositif de mesure d'un effort exercé par un fémur sur un tibia en vue d'une pose de prothèse articulaire, le dispositif étant notamment destiné à être agencé sous un tenseur qui écarte le tibia du fémur, le dispositif comportant :
- un substrat ayant une première face destinée à être posée sur une surface du tibia pour recouvrir ladite surface;
- un réseau d'entrée, un premier réseau de sortie et un deuxième réseau de sortie électriquement conducteurs, chaque réseau comportant des branches parallèles qui sont reliées entre elles à la manière d'un peigne, lesdits réseaux s'étendant sur une deuxième face du substrat opposée à la première face de sorte qu'une première partie des branches du réseau d'entrée s'étendent en alternance avec les branches du premier réseau de sortie pour s'étendre en service sous un premier condyle du fémur, et qu'une deuxième partie des branches du réseau d'entrée s'étendent en alternance avec les branches du deuxième réseau de sortie pour s'étendre en service sous un deuxième condyle du fémur ;
- un coussin en matériau élastiquement déformable et électriquement conducteur recouvrant la deuxième face du substrat.

Ainsi, le coussin se déforme localement en fonction de l'effort exercé par le condyle concerné sur le tibia. Plus le coussin est compressé, plus il favorise un passage d'un courant électrique entre les branches du réseau d'entrée et les branches du réseau de sortie associé. Il est donc possible de cartographier avec précision l'effort exercé par chacun des condyles sur le tibia en mesurant une tension soit entre le réseau d'entrée et le premier réseau de sortie, soit entre le réseau d'entrée et le deuxième réseau de sortie. Trois fils suffisent donc pour obtenir ces deux informations.

Le dispositif selon l'invention ne nécessite aucun élément complexe pour caractériser l'effort exercé à l'articulation du genou par le fémur sur le tibia. En outre, le dispositif ne nécessite que trois fils de raccordement (un pour chaque réseau) grâce au réseau d'entrée commun.

Le dispositif selon l'invention s'avère particulièrement peu encombrant puisque l'on se passe de plaque rigide pour appuyer directement sur le coussin. Tout le dispositif repose à l'interstice entre fémur et tibia à l'exception des fils raccordant les réseaux. Les inventeurs ont ainsi pu élaborer un prototype de moins de 2 millimètres d'épaisseur.

### BREVE DESCRIPTION DES DESSINS

L'invention sera mieux comprise à la lumière de la description qui suit d'un mode de réalisation particulier non limitatif de l'invention.

Il sera fait référence aux figures ci-jointes, parmi lesquelles :
- la figure 1 est une vue éclatée schématique d'un dispositif de mesure selon l'invention ;
- la figure 2 est une vue du dessus du substrat du dispositif de mesure illustré à la figure 1 ;
- la figure 3 est une vue schématique en perspective du dispositif de mesure selon l'invention disposé en service à l'articulation d'un genou.

### DESCRIPTION DETAILLEE DE L'INVENTION

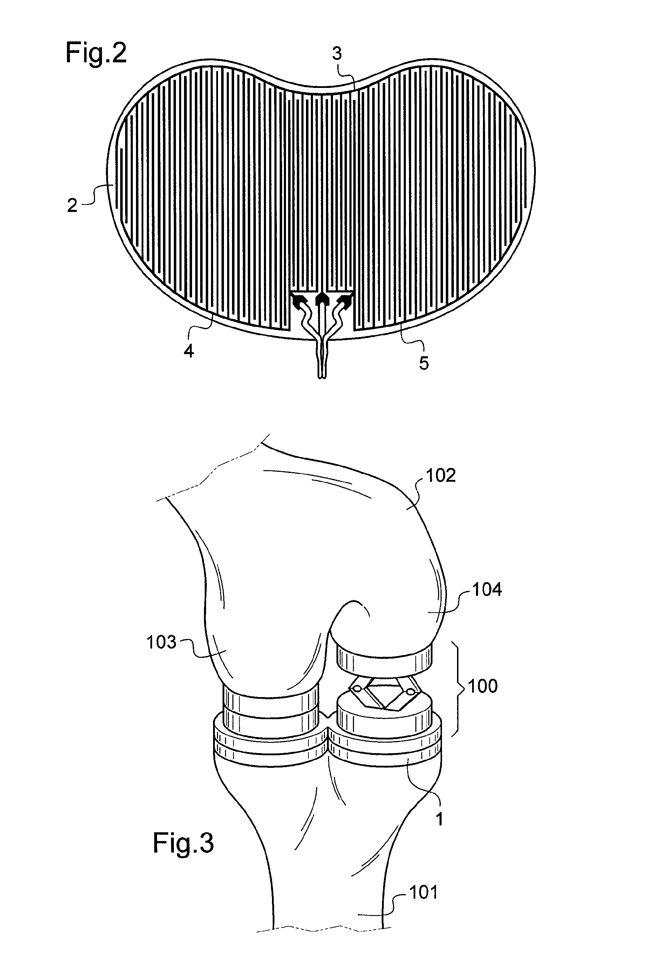

En référence à la figure 3, le dispositif de mesure selon l'invention est destiné à être agencé sous un tenseur 100 qui écarte le tibia 101 du fémur 102. De tels tenseurs sont bien connus, le tenseur 100 ne sera donc pas décrit plus en détails ici. Le tenseur illustré permet ici d'écarter ou de rapprocher indépendamment chaque condyle du fémur 102 du tibia 101. Cet exemple de tenseur n'est bien sûr pas limitatif et le dispositif de mesure pourra être disposé en service sous n'importe quel type de tenseur.

Ici, le tibia 101 a été coupé et les condyles du fémur 102 partiellement coupés en vue d'une pose de prothèse articulaire. Puis, un chirurgien a introduit le tenseur 100 à l'articulation du genou ainsi que le dispositif de mesure de l'invention.

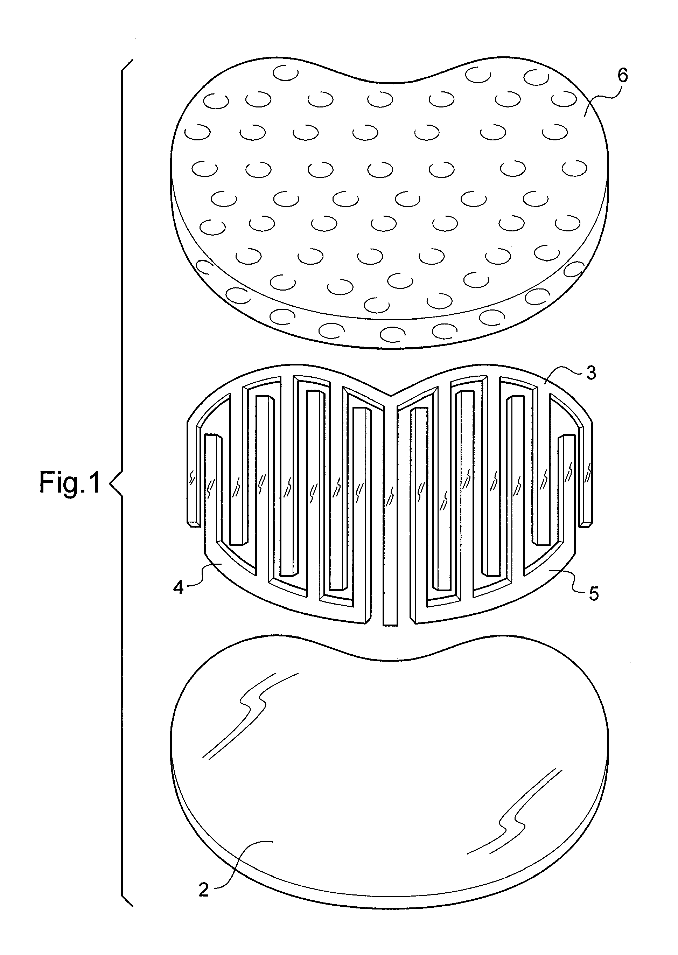

En référence aux figures 1 à 3, le dispositif de mesure 1 comporte un substrat 2 ayant une première face posée en service sur la portion coupée du tibia 101 pour recouvrir ladite portion.

Le dispositif de mesure comporte également un réseau d'entrée 3, un premier réseau de sortie 4 et un deuxième réseau de sortie 5 électriquement conducteurs, chaque réseau 3, 4, 5 comportant des branches parallèles qui sont reliées entre elles à la manière d'un peigne. Les réseaux 3, 4, 5 s'étendent sur une deuxième face du substrat 2 opposée à la première face de sorte qu'une première partie des branches du réseau d'entrée 3 s'étendent parallèlement et en alternance avec les branches du premier réseau de sortie 4 pour s'étendre en service sous un premier condyle 103 du fémur 102. Identiquement, une deuxième partie des branches du réseau d'entrée 3 s'étendent parallèlement et en alternance avec les branches du deuxième réseau de sortie 5 de sorte à s'étendre en service sous un deuxième condyle 104 du fémur 102. Selon un mode particulier de réalisation, le substrat 2 est une carte de type à circuits imprimés, les réseaux étant réalisés par des techniques classiques de photo-impression et attaque chimique. D'autres réalisations pourront être envisagées sans sortir du cadre de l'invention.

Le dispositif de mesure comporte en outre un coussin 6 en matériau élastiquement déformable et électriquement conducteur qui recouvre la deuxième face du substrat 2. Ici, le coussin 6 comporte une couche en mousse chargée en particules de carbone qui vient directement en contact avec les branches des réseaux 3, 4, 5.

Le dispositif est ainsi conformé de sorte à recouvrir entièrement la portion coupée du tibia 101.

Selon un mode de réalisation privilégié, comme illustré à la figure 3, le substrat 2, le réseau d'entrée 3, les deux réseaux de sortie 4, 5 et le coussin 6 recouvrant la deuxième face du substrat 2 forment un ensemble unitaire 1 encapsulé ici dans une enveloppe en silicone déformable laissant uniquement sortir trois fils connectant chaque réseau à un organe de traitement de données(non illustré ici).

En service, le chirurgien décale le fémur 102 par rapport au tibia 101. Le coussin 6 est alors écrasé localement selon l'effort exercé par les condyles du fémur 102 sur le tibia 101. Plus le coussin 6 est compressé plus il favorise un passage d'un courant électrique entre les branches du réseau d'entrée 3 et les branches du réseau de sortie associé. L'organe de traitement mesure alors une tension entre le réseau d'entrée 3 et le premier réseau de sortie 4 ainsi qu'entre le réseau d'entrée 3 et le deuxième réseau de sortie 5 pour caractériser l'effort exercé par chacun des condyles 103, 104 sur le tibia 101. Grâce à l'agencement avantageux des branches des réseaux 3, 4, 5, trois fils suffisent pour que le dispositif puisse réaliser cette comparaison.

Le chirurgien peut alors, à partir des mesures obtenues pour différents angle de flexion du genou, déterminer précisément les dimensions de la prothèse adaptée au patient et affiner ici la coupe du fémur 102 de sorte à équilibrer l'effort exercé par chacun des condyles 103, 104 sur le tibia 101 et ce que le genou soit fléchi ou en extension. Il peut également utiliser ces mesures en temps réel pour régler le tenseur de sorte à équilibrer les tensions des ligaments liant le fémur 102 au tibia 101.

L'invention n'est pas limitée à ce qui vient d'être décrit et englobe toute variante entrant dans le cadre défini par les revendications.

## Revendications

1. Dispositif de mesure d'un effort exercé par un fémur (102) sur un tibia (101) en vue d'une pose de prothèse articulaire, le dispositif étant notamment destiné à être agencé sous un tenseur (100) qui écarte le tibia du fémur, le dispositif comportant :
- un substrat (2) ayant une première face destinée à être posée sur une surface du tibia pour recouvrir ladite surface;
- un réseau d'entrée (3), un premier réseau de sortie (4) et un deuxième réseau de sortie (5) électriquement conducteurs, chaque réseau comportant des branches parallèles qui sont reliées entre elles à la manière d'un peigne, lesdits réseaux s'étendant sur une deuxième face du substrat opposée à la première face de sorte qu'une première partie des branches du réseau d'entrée s'étendent en alternance avec les branches du premier réseau de sortie pour s'étendre en service sous un premier condyle (103) du fémur, et qu'une deuxième partie des branches du réseau d'entrée s'étendent en alternance avec les branches du deuxième réseau de sortie pour s'étendre en service sous un deuxième condyle (104) du fémur ;
- un coussin en matériau (6) élastiquement déformable et électriquement conducteur recouvrant la deuxième face du substrat.

2. Dispositif de mesure selon la revendication 1, dans lequel le coussin (6) recouvrant la deuxième face du substrat (2) comporte une couche en mousse chargée en particules de carbone qui vient directement en contact avec les branches des réseaux.

3. Dispositif de mesure selon la revendication 1, dans lequel le substrat (2), le réseau d'entrée (3), les deux réseaux de sortie (4,5) et le coussin (6) recouvrant la deuxième face du substrat forment un ensemble unitaire (1) .

4. Dispositif de mesure selon la revendication 3, dans lequel le dispositif est encapsulé dans une enveloppe en silicone déformable.

5. Dispositif de mesure selon la revendication 1, dans lequel les branches des réseaux sont imprimées sur le substrat (2).
